# EUROPEAN PATENT APPLICATION

(11) **EP 3 804 669 A1**
(43) Date of publication of application: **14.04.2021**
(21) Application number: 18921096.6
(22) Date of filing: 29.05.2018
(51) Int. Cl.: A61F 9/007, A61F 9/008

(54) **INTRAOCULAR ILLUMINATION DEVICE**

(71) Applicant: Neuroceuticals Inc., Tokyo 113-0033 (JP)
(72) Inventor: MIIKE, Shinya, Tokyo 113-0033 (JP)
(74) Representative: Mewburn Ellis LLP
(86) International application number: PCT/JP2018/020521
(87) International publication number: WO 2019/229840

(57) **Abstract**

An intraocular lighting device that is inserted into an eye in order to illuminate the inside of the eye includes a fiber-shaped light-guiding unit for guiding light emitted from a light source to an emission end, and a scattering unit that is optically connected to the light-guiding unit at the emission end and contains a predetermined scattering medium for scattering the light emitted from the light-guiding unit, a dimension of the scattering unit in a parallel direction to an optical axis direction being set to be longer than a dimension in a perpendicular direction to the optical axis direction, and the predetermined scattering medium scattering light to a larger degree as the wavelength of the light shortens.

## Description

### Technical Field

The present invention relates to an intraocular lighting device.

### Background Art

Important slight-related organs, such as the retina and photoreceptor cells (cone cells and rod cells) are formed in the ocular fundus of humans and animals. When surgery is performed on the ocular fundus in which these are organs are positioned, an intraocular lighting probe is used to illuminate the inside of the eye. The intraocular lighting probe illuminates the inside of the eye by guiding illumination laser light supplied from a light source so that the illumination laser light is emitted from an emission end portion disposed inside the eye.

Light emitted from a light guide such as an optical fiber used in an intraocular lighting probe often has high directivity in a front direction of the optical axis. The body receives considerable damage when irradiated with light, and therefore the importance of protecting the ocular fundus, in which important sight-related organs are formed, from the illumination laser light is particularly high in comparison with other positions inside the eye.

Regarding this point, Patent Document 1, for example, describes a lighting probe having an optical fiber with a tapered end portion. In this lighting probe, by tapering the end portion of the optical fiber, the angular distribution of the illumination laser light emitted from the lighting probe is widened, thereby reducing a component of the light that is emitted toward the ocular fundus positioned in the optical axis direction, and as a result, the ocular fundus can be protected to a certain extent.

### Citation List

### Patent Document

Patent Document 1: Patent Publication JP-A-2014-512851

### Summary

### Technical Problem

In the lighting probe described in Patent Document 1, however, the light components contained in the illumination laser light, despite having different wavelengths, exhibit similarities in the manner in which the angular distributions thereof widen. Therefore, when the illuminance of the illumination laser light is adjusted to a point at which the ocular fundus can be sufficiently protected using a light component having a short wavelength, which causes great damage to the body, as a reference, the resulting illuminance may not be sufficient for the surgeon.

An object of the present invention is therefore to provide an intraocular lighting device with which the ocular fundus can be sufficiently protected while maintaining high illuminance.

### Solution to Problem

An intraocular lighting device according to an aspect of the present invention is inserted into an eye in order to illuminate the inside of the eye, and includes a fiber-shaped light-guiding unit for guiding light emitted from a light source to an emission end, and a scattering unit that is optically connected to the light-guiding unit at the emission end and contains a predetermined scattering medium for scattering the light emitted from the light-guiding unit, a dimension of the scattering unit in a parallel direction to an optical axis direction being set to be longer than a dimension in a perpendicular direction to the optical axis direction, and the predetermined scattering medium scattering light to a larger degree as the wavelength of the light shortens.

According to this aspect, as an angular difference between the optical path and the optical axis decreases, the distance light travels through the scattering unit basically increases, leading to an increase in the probability of the light being scattered by the scattering medium, which scatters light to a larger degree as the wavelength of the light shortens. Thus, the angular distribution of short-wavelength light can be selectively widened, and as a result, the ocular fundus can be sufficiently protected while maintaining high illuminance.

### Advantageous Effects of Invention

According to the present invention, it is possible to provide an intraocular lighting device with which the ocular fundus can be sufficiently protected while maintaining high illuminance.

### Brief Description of Drawings

Fig. 1 is a view showing an example configuration of an intraocular lighting system 1 according to an embodiment.
Fig. 2 is a perspective view showing an example configuration of an intraocular lighting device 10.
Fig. 3 is a schematic sectional view taken along an A-A' line in Fig. 2.
Fig. 4 is a view showing an example of an irradiance distribution of light emitted by the intraocular lighting device 10.
Fig. 5 is a view showing an example of a color temperature distribution of the light emitted by the intraocular lighting device 10.

### Description of Embodiments

Referring to the attached figures, a preferred embodiment of the present invention will be described (note that in the figures, identical reference numerals denote identical or similar configurations).

### (1) Configuration

### (1-1) Intraocular lighting system 1

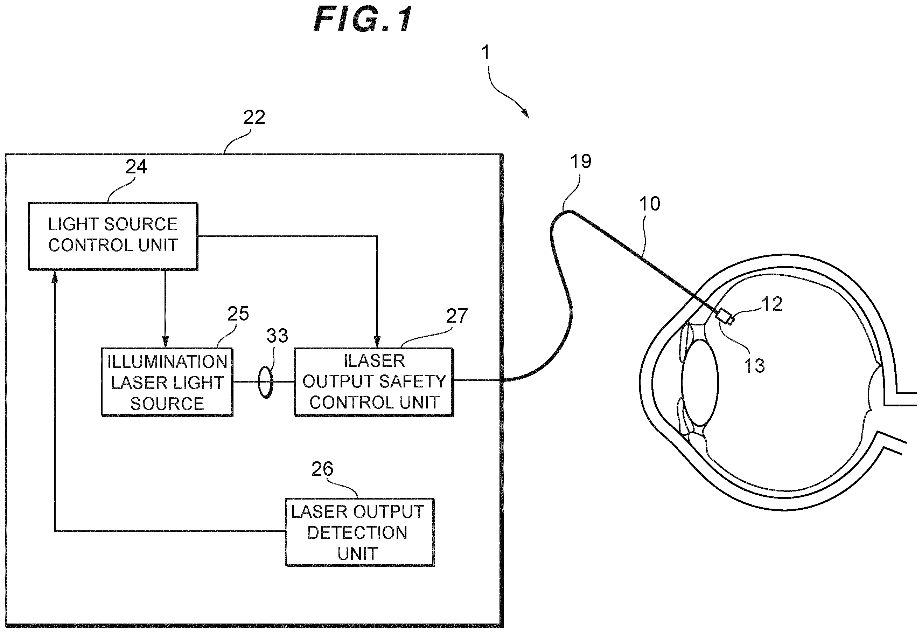

Fig. 1 is a view showing an example configuration of an intraocular lighting system 1 according to an embodiment. The intraocular lighting system 1 is a system for illuminating the inside of the eye when surgery is performed on the ocular fundus using a surgical probe or the like, not shown in the figure. The intraocular lighting system 1 includes a light source unit 22 and an intraocular lighting device 10 that is optically connected to the light source unit 22.

### (1-2) Light source unit 22

The light source unit 22 supplies the intraocular lighting device 10 with illumination light. The light source unit 22 includes, for example, a light source control unit 24, an illumination laser light source 25, a laser output detection unit 26, a laser output safety control unit (a calibration device) 27, and a condenser lens 33. Note that the illumination light supplied by the light source unit 22 is not limited to laser light and may be any other type of light.

The light source control unit 24 controls the overall operation of the light source unit 22 in order to control light emission by the light source.

The illumination laser light source 25 generates illumination laser light to be supplied to the intraocular lighting device 10. There are no particular limitations on the type of illumination laser light, but for example, the illumination laser light source 25 may generate light having a wavelength that corresponds to a contrast medium used in a fundus fluorescein angiography method. Alternatively, the illumination laser light source 25 may generate light having wavelengths in a visible region, such as white light, for example.

The laser output detection unit 26 detects the luminous intensity of the illumination laser light generated by the illumination laser light source 25. On the basis of a detection result from the laser output detection unit 26, the laser output safety control unit 27 makes the irradiation intensity of the illumination laser light on the surface of the ocular fundus constant.

The condenser lens 33 is disposed between the illumination laser light source 25 and the laser output safety control unit 27.

### (1-3) Intraocular lighting device 10

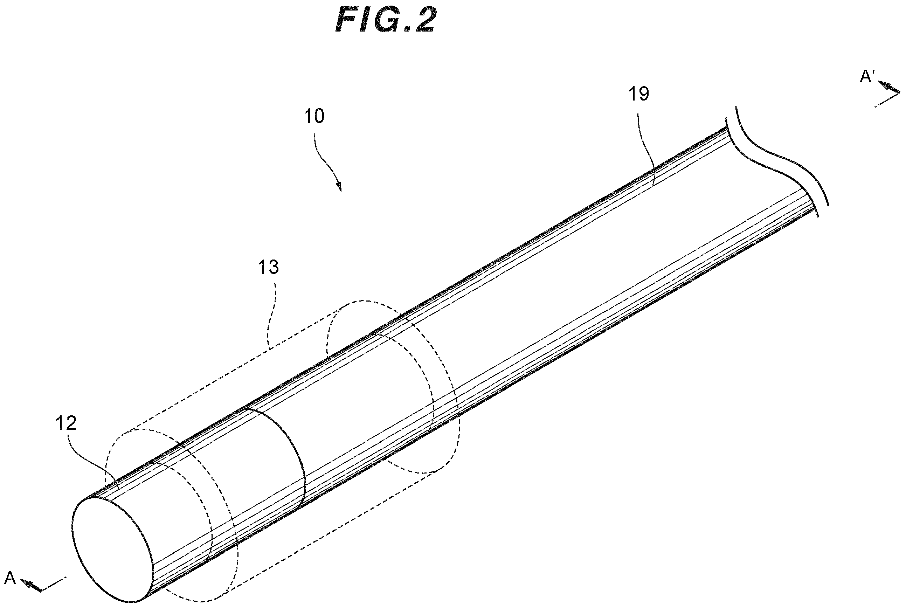

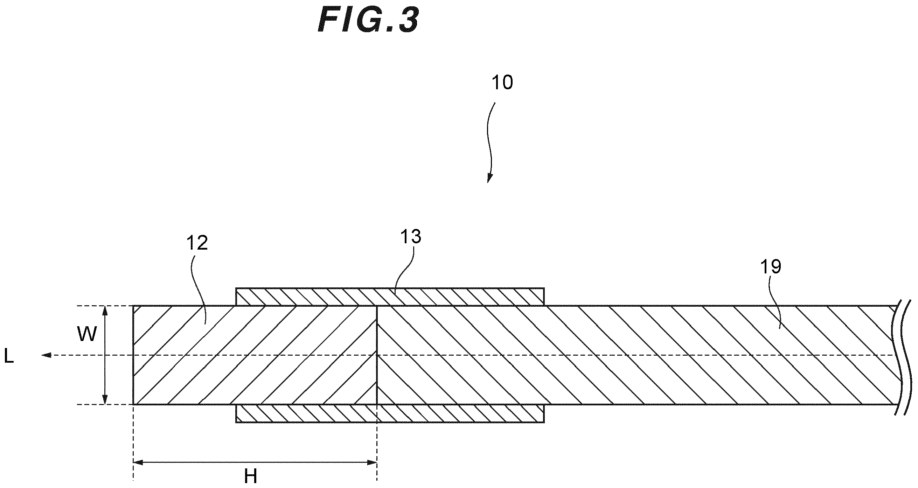

Fig. 2 is a perspective view showing an example configuration of the intraocular lighting device 10. Fig. 3 is a schematic sectional view taken along an A-A' line in Fig. 2. Note that in Fig. 2, a protective unit 13, to be described below, is illustrated in a transparent state.

The intraocular lighting device 10 can be inserted into the eye, and illuminates the inside of the eye by receiving a supply of illumination laser light from the light source unit 22 through an illumination light-guiding fiber 19 and emitting the illumination laser light from an emission end disposed inside the eye. As shown in Fig. 2, the intraocular lighting device 10 includes the illumination light-guiding fiber 19, a scattering unit 12, and the protective unit 13.

The illumination light-guiding fiber 19 is constituted by an optical fiber having a substantially cylindrical shape, for example. One end of the illumination light-guiding fiber 19 serves as an entrance end for the illumination laser light, and the other end serves as the emission end. The entrance end of the illumination light-guiding fiber 19 is optically connected to the light source unit 22, while the emission end of the illumination light-guiding fiber 19 is optically connected to the scattering unit 12. The illumination laser light that enters the entrance end of the illumination light-guiding fiber 19 from the light source unit 22 propagates through the interior of the illumination light-guiding fiber 19 to the emission end and is then emitted from the emission end so as to enter the scattering unit 12.

The scattering unit 12 is formed in a substantially cylindrical shape from translucent resin and contains a predetermined scattering medium, for example. One end of the scattering unit 12 serves as an entrance end for the illumination laser light. The illumination laser light that enters the entrance end of the scattering unit 12 from the illumination light-guiding fiber 19 is emitted to the exterior of the scattering unit 12 while being partially scattered in arbitrary directions by the scattering medium contained in the scattering unit 12.

A scattering medium having a property whereby the degree to which light is scattered increases as the wavelength of the light shortens, for example, is preferably used as the predetermined scattering medium contained in the scattering unit 12. The predetermined scattering medium may be a fluorescent pigment, for example. Further, the scattering unit 12 may contain a plurality of types of scattering media. Furthermore, the scattering medium content of the scattering unit 12 may be uniform over the entire scattering unit 12, or the scattering medium content of the scattering unit 12 may be non-uniform over the entire scattering unit 12. The scattering medium content of the scattering unit 12 may be distributed so as to have a predetermined gradient along an optical axis L or another direction or may differ between the side of the illumination light-guiding fiber 19 and the opposite side to the illumination light-guiding fiber 19, for example.

The protective unit 13 is formed in a substantially cylindrical shape from translucent resin, synthetic silica glass, or the like, for example, and has a through hole that extends in the axial direction. The protective unit 13 protects at least a part of the illumination light-guiding fiber 19 and at least a part of the scattering unit 12 along the optical axis L. The illumination laser light scattered by the scattering unit 12 passes through the protective unit 13.

### (2) Dimensions of scattering unit 12

Here, using Fig. 3, the dimensions of the scattering unit 12 will be described. In Fig. 3, a dotted-line arrow indicates the optical axis L of the illumination light-guiding fiber 19.

As shown in Fig. 3, a dimension of the scattering unit 12 in a parallel direction to the optical axis L is set as H, and a dimension of the scattering unit 12 in a perpendicular direction to the optical axis L is set as W. At this time, the dimensions of the scattering unit 12 are set so that H is longer than W. Thus, as the angular difference between the optical path and the optical axis L decreases, the distance light travels through the scattering unit 12 basically increases, leading to an increase in the probability of the illumination laser light being scattered by the scattering medium contained in the scattering unit 12.

### (3) Distribution of emitted light

### (3-1) Irradiance distribution of emitted light

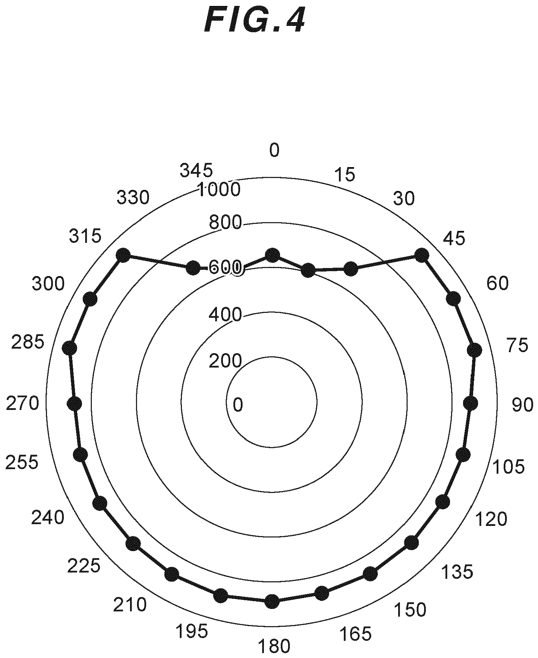

Fig. 4 is a view showing an example of an irradiance distribution of the light emitted by the intraocular lighting device 10. In Fig. 4, a circumferential direction axis shows the emission angle of the emitted light using the optical axis L as a reference, while a radial direction axis shows the irradiance (unit: µW/cm²) of the emitted light.

As shown in Fig. 4, the irradiance of the emitted light within a range of 45 to 315 degrees is approximately 900 µW/cm². Further, the radiant intensity of the emitted light within a range of 315 to 45 degrees basically decreases toward 0 degrees and is approximately 600 µW/cm² in the vicinity of 0 degrees.

Hence, in the intraocular lighting device 10 according to this embodiment, the dimension H of the scattering unit 12 in a parallel direction to the optical axis L is longer than the dimension W of the scattering unit 12 in a perpendicular direction to the optical axis L, with the result that the illuminance of the illumination laser light emitted in the front direction of the intraocular lighting device 10 decreases. Therefore, with the intraocular lighting device 10 according to this embodiment, the inside of the eye can be illuminated more evenly, and the retina and so on positioned in the front direction of the intraocular lighting device 10 can be protected appropriately from the illumination laser light.

### (3-2) Color temperature distribution of emitted light

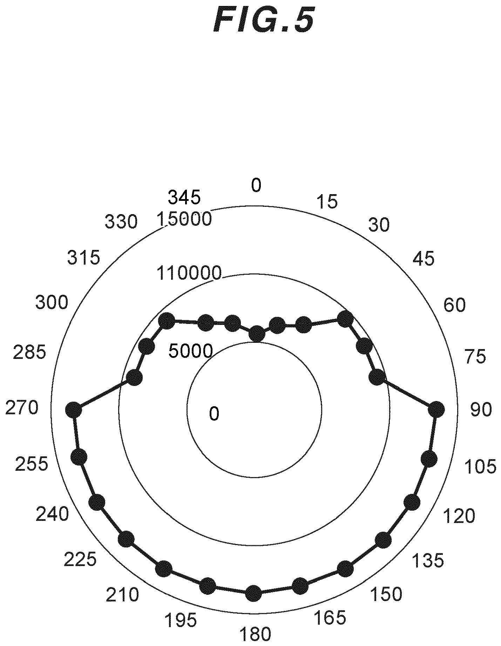

Fig. 5 is a view showing an example of a color temperature distribution of the light emitted by the intraocular lighting device 10. In Fig. 5, a circumferential direction axis shows the emission angle of the emitted light using the optical axis L as a reference, while a radial direction axis shows the color temperature (unit: K) of the emitted light. Here, the color temperature is a measure of the color of the light, which varies in accordance with the distribution of the wavelengths contained in the light. When the color temperature is high, the light contains a relatively large amount of short-wavelength light, and as a result, the light has a bluish tinge. When the color temperature is low, the light contains a relatively large amount of long-wavelength light, and as a result, the light has a reddish tinge.

As shown in Fig. 5, the color temperature of the emitted light within a range of 90 to 270 degrees is approximately 13000 to 14000 K. Further, the radiant intensity of the emitted light within a range of 270 to 90 degrees decreases toward 0 degrees and is approximately 5000 K in the vicinity of 0 degrees.

Hence, in the intraocular lighting device 10 according to this embodiment, the scattering unit 12 contains a scattering medium that scatters light to a greater degree as the wavelength of the light shortens, and therefore the effect of setting the dimensions of the scattering unit 12 so that H > W, as described above, increases as the wavelength of the light shortens. As a result, the proportion of short-wavelength light contained in the illumination laser light emitted in the front direction of the intraocular lighting device 10 decreases (the color temperature of the illumination laser light decreases). Hence, with the intraocular lighting device 10 according to this embodiment, the ocular fundus (the important sight-related organs) positioned in the front direction of the intraocular lighting device 10 can be protected selectively from the short-wavelength light contained in the illumination laser light while maintaining an even color temperature as a whole.

### (4) Miscellaneous

In Fig. 5, the average color temperature generated inside the eye by the intraocular lighting device 10 may be set at approximately 6000 K ± 1000 K, for example. This color temperature is close to natural light, and therefore an operator can easily check the ocular fundus.

The embodiment described above is to be used to facilitate understanding of the present invention, and the present invention is not limited thereto. The elements included in the embodiment, as well as the arrangements, materials, conditions, shapes, sizes, and so on thereof, are not limited to the cited examples and may be modified as appropriate. Moreover, configurations illustrated in different embodiments may be partially replaced or combined.

### Reference Signs List

- 1: Intraocular lighting system
- 10: Intraocular lighting device
- 19: Illumination light-guiding fiber
- 22: Light source unit
- 24: Light source control unit
- 25: Illumination laser light source
- 26: Laser output detection unit
- 27: Laser output safety control unit
- 33: Condenser lens

## Claims

1. An intraocular lighting device that is inserted into an eye in order to illuminate the inside of the eye, comprising:
a fiber-shaped light-guiding unit for guiding light emitted from a light source to an emission end; and
a scattering unit that is optically connected to the light-guiding unit at the emission end and contains a predetermined scattering medium for scattering the light emitted from the light-guiding unit, a dimension of the scattering unit in a parallel direction to an optical axis direction being set to be longer than a dimension in a perpendicular direction to the optical axis direction, and the predetermined scattering medium scattering light to a larger degree as a wavelength of the light shortens.

2. The intraocular lighting device according to claim 1, wherein the scattering unit has a substantially columnar shape with a parallel axis to the optical axis direction.

3. The intraocular lighting device according to claim 2, wherein the scattering unit has a substantially cylindrical shape with a parallel axis to the optical axis direction.

4. The intraocular lighting device according to any one of claims 1 to 3, wherein the predetermined scattering medium is a fluorescent pigment.

5. The intraocular lighting device according to any one of claims 1 to 4, wherein the dimension of the scattering unit in a parallel direction to the optical axis direction and the dimension of the scattering unit in a perpendicular direction to the optical axis direction are set so that an average color temperature inside the eye is approximately 6000 K ± 1000 K.

## Amended claims

### Amended claims under Art. 19.1 PCT

1. (Amended) An intraocular lighting device that is inserted into an eye in order to illuminate the inside of the eye, comprising:
a fiber-shaped light-guiding unit for guiding light emitted from a light source to an emission end; and
a scattering unit that is optically connected to the light-guiding unit at the emission end and contains a predetermined scattering medium for scattering the light emitted from the light-guiding unit in arbitrary directions inside the eye, a dimension of the scattering unit in a parallel direction to an optical axis direction being set to be longer than a dimension in a perpendicular direction to the optical axis direction, and the predetermined scattering medium scattering light to a larger degree as a wavelength of the light shortens.

2. The intraocular lighting device according to claim 1, wherein the scattering unit has a substantially columnar shape with a parallel axis to the optical axis direction.

3. The intraocular lighting device according to claim 2, wherein the scattering unit has a substantially cylindrical shape with a parallel axis to the optical axis direction.

4. The intraocular lighting device according to any one of claims 1 to 3, wherein the predetermined scattering medium is a fluorescent pigment.

5. The intraocular lighting device according to any one of claims 1 to 4, wherein the dimension of the scattering unit in a parallel direction to the optical axis direction and the dimension of the scattering unit in a perpendicular direction to the optical axis direction are set so that an average color temperature inside the eye is approximately 6000 K ± 1000 K.
